# EUROPEAN PATENT APPLICATION

(11) **EP 4 202 053 A1**
(43) Date of publication of application: **28.06.2023**
(21) Application number: 20950062.8
(22) Date of filing: 29.10.2020
(51) Int. Cl.: C12P 19/34, C07H 21/02

(54) **NOVEL 5'CAP ANALOG HAVING CAP2 STRUCTURE AND PREPARATION METHOD THEREFOR**

(30) Priority: 20.08.2020 CN 202010842263
(71) Applicant: Shenzhen Rhegen Biotechnology Co., Ltd., Shenzhen, Guangdong 518057 (CN)
(72) Inventor: HU, Yong, Shenzhen, Guangdong 518057 (CN); ZHANG, Miaomiao, Shenzhen, Guangdong 518057 (CN); HONG, Dan, Shenzhen, Guangdong 518057 (CN); HU, Xun, Shenzhen, Guangdong 518057 (CN)
(74) Representative: Engelhard, Markus
(86) International application number: PCT/CN2020/124720
(87) International publication number: WO 2022/036858

(57) **Abstract**

A 5'Cap analog having a Cap2 structure and a preparation method therefor. The molecular formula of the 5'Cap analog having a Cap2 structure is selected from any one of m7G(5')ppp(5')(2'OMeA)p(2'OMeG), m7G(5')ppp(5')(2'OMeG)p(2'OMeG), etc. The 5'Cap analog having a Cap2 structure has higher synthesis efficiency, higher capping efficiency, lower immunogenicity, and higher protein translation efficiency.

## Description

This application claims priority to a Chinese patent application filed with the Chinese Patent Office on August 20, 2020, Application No. 202010842263.2, entitled "Novel 5'Cap analog having Cap2 structure and preparation method therefor". The entire contents of which are incorporated by reference in this application.

### Field of the invention

The invention generally relates to the field of genetic engineering technology, and specifically, to a novel 5'Cap analog having a Cap2 structure and its preparation method.

### Background of the invention

Translation efficiency and immunogenic are two key factors for mRNA drugs to exercise their functions. Autoimmune proteins such as RIG-I and IFIT recognize the mRNAs with abnormal cap, reducing the intracellular activity and half-life of exogenous mRNAs, making it difficult for mRNA drugs to function in vivo. Therefore, optimizing the mRNA cap structure is essential to improve the biological activity and reduce the immunogenicity of mRNAs.

There are currently two methods to synthesize mRNA with cap structure in vitro. In the first method, after mRNA is transcribed, it is capped with Vaccinia Virus Capping Enzyme to produce RNA with Cap 0 structure or Cap 1 structure. This method is efficient, but the yield is unstable and expensive. In addition, the enzymatic capping method fails to produce the caps with Cap 2 structure and m6Am structure. The second method is to add an excess amount of Cap analogs for capping during transcription. By far the most commonly used structural Cap analog is ARCA, using which immunogenic Cap 0 can be produced, but with a capping efficiency of only 70%. The yield of this method is also relatively low, with a maximum of 1.5 mg RNA prepared per ml of transcription reaction system, and the transcribed product is highly immunogenic. Another novel co-transcriptional capping method called Cleancap is also widely used. Cleancap belongs to Cap 1, and unlike ARCA which uses a dimer (m7GpppG) to initiate T7 transcription, CleanCap uses a trimer (m7GpppAmG) to initiate T7 transcription. The method has a relatively high yield, preparing 4 mg of capped RNA per ml of transcription reaction system with a capping efficiency of 90%, and its transcription products are less immunogenic than ARCA.

Based on the drawbacks of low capping efficiency, low mRNA yield per synthesis volume and high immunogenicity of the existing Cap analogs in mRNA synthesis system, the inventors developed a novel Cap analog having a Cap2 structure, which not only compensates for the above application drawbacks but also greatly improves the protein translation efficiency.

### Summary of the invention

Based on the above reasons, the present invention provides a novel 5'Cap analog having a Cap2 structure and its preparation method. In addition, the novel 5'Cap analog having a Cap2 structure provided by the present invention presents higher synthesis efficiency, higher capping efficiency, lower immunogenicity and higher protein translation efficiency.

The present invention is realized by the following technical solutions.

A novel 5'Cap analog having a Cap2 structure, the novel 5'Cap analog having a Cap2 structure has a molecular formula selected from any one of the following formulae:
m7G(5')ppp(5')(2'OMeA)p(2'OMeG),
m7G(5')ppp(5')(2'OMeG)p(2'OMeG),
m7G(5')ppp(5')(2'OMeC)p(2'OMeG),
m7G(5')ppp(5')(2'OMeU)p(2'OMeG),
m7G(5')ppp(5')(2'OMeA)p(2'OMeA),
m7G(5')ppp(5')(2'OMeG)p(2'OMeA),
m7G(5')ppp(5')(2'OMeC)p(2'OMeA),
m7G(5')ppp(5')(2'OMeU)p(2'OMeA),
m7G(5')ppp(5')(2'OMeA)p(2'OMeC),
m7G(5')ppp(5')(2'OMeG)p(2'OMeC),
m7G(5')ppp(5')(2'OMeC)p(2'OMeC),
m7G(5')ppp(5')(2'OMeU)p(2'OMeC),
m7G(5')ppp(5')(2'OMeA)p(2'OMeU),
m7G(5')ppp(5')(2'OMeG)p(2'OMeU),
m7G(5')ppp(5')(2'OMeC)p(2'OMeU),
m7G(5')ppp(5')(2'OMeU)p(2'OMeU),
3'-O-Me-m7G(5')ppp(5')(2'OMeA)p(2'OMeG),
3'-O-Me-m7G(5')ppp (5')(2'OMeG)p(2'OMeG),
3'-O-Me-m7G(5')ppp(5')(2'OMeC)p(2'OMeG),
3'-O-Me-m7G(5')ppp(5')(2'OMeU)p(2'OMeG),
3'-O-Me-m7G(5')ppp(5')(2'OMeA)p(2'OMeA),
3'-O-Me-m7G(5')ppp(5')(2'OMeG)p(2'OMeA),
3'-O-Me-m7G(5')ppp(5')(2'OMeC)p(2'OMeA),
3'-O-Me-m7G(5')ppp(5')(2'OMeU)p(2'OMeA),
3'-O-Me-m7G(5')ppp(5')(2'OMeA)p(2'OMeC),
3'-O-Me-m7G(5')ppp(5')(2'OMeG)p(2'OMeC),
3'-O-Me-m7G(5')ppp(5')(2'OMeC)p(2'OMeC),
3'-O-Me-m7G(5')ppp(5')(2'OMeU)p(2'OMeC),
3'-O-Me-m7G(5')ppp(5')(2'OMeA)p(2'OMeU),
3'-O-Me-m7G(5')ppp(5')(2'OMeG)p(2'OMeU),
3'-O-Me-m7G(5')ppp(5')(2'OMeC)p(2'OMeU), and
3'-O-Me-m7G(5')ppp(5')(2'OMeU)p(2'OMeU).

The present invention further provides a method for the preparation of a novel 5'Cap analog having a Cap2 structure as described in the above technical solution, comprising the steps of
(1) 2'-O-Methyl-ATP, 2'-O-Methyl-GTP, 2'-O-Methyl-CTP and 2'-O-Methyl-UTP were dissolved in RNase-free water respectively, mixed with phosphate hydrolase and 2× Reaction Buffer respectively, and followed by incubation to obtain 2'-O-Methyl-ADP, 2'-O-Methyl-GDP, 2'-O-Methyl-CDP and 2'-O-Methyl-UDP;
(2) The 2'-O-Methyl-ADP, 2'-O-Methyl-GDP, 2'-O-Methyl-CDP and 2'-O-Methyl-UDP obtained from the step 1) were dissolved in RNase-free water, mixed with 7-Methylguanosine, 7-Methyl-3'-O Methylguanosine, guanosyltransferase, and 2× Reaction Buffer respectively, and followed by incubation to obtain m7G(5')ppp(5')(2'OMeA/G/C/LT) and 3'-O-Me-m7G(5')ppp(5')(2'OMeA/G/C/LT); and
3) The m7G(5')ppp(5')(2'OMeA/G/C/U) and 3'-O-Me-m7G(5')ppp(5')(2'OMeA/G/C/U) obtained from the step 2) were dissolved in RNase-free water respectively, mixed with T4 RNALigase1 and then mixed with 2'-O-Methyl-ATP, 2'-O-Methyl-GTP, 2'-O-Methyl-CTP and 2'-O-Methyl-UTP respectively, followed by mixing with 2×T4 RNA Ligase Reaction Buffer and incubation to obtain the novel 5'Cap analog having a Cap2 structure.

Preferably, the ratio of the amount of 2'-O-methyl-ATP, 2'-O-methyl-GTP, 2'-O-methyl-CTP, and 2'-O-methyl-UTP in the step 1) to the volume of RNase-free water is 1 to 30 mmol:14 µL.

Preferably, the volume ratio of RNase-free water to phosphate hydrolase and 2× Reaction Buffer in the step 1) is 14:1:15.

Preferably, the phosphate hydrolase has an enzymatic activity of 50,000 U.

The components of the 2× Reaction Buffer comprise: 50 mM Tris-HCl, 5 mM KCl, 1 mM MgCl₂, and 1 mM DTT, with pH value of 8.

Preferably, the incubation temperature of the steps 1) to 3) is 37 °C respectively, and the incubation time thereof is 1h respectively.

Preferably, in the step 2), the volume ratio of RNase-free water to 7-methyl-guanosine, 7-methyl-3 '-O-methyl-guanosine, guanosyltransferase and 2× reaction buffer is 11:10:10:1:22.

Preferably, in the step 3), the volume ratio of RNase-free water to T4 RNA ligase 1 and 2× T4 RNA ligase reaction buffer is 11:1:22.

Preferably, the components of the 2×T4 RNA Ligase Reaction Buffer comprise: 60 mM Tris-HCl, 20 mM MgCl₂, 20 mM DTT and 2 mM ATP.

Preferably, the enzyme activity of the T4 RNA Ligase 1 is 10,000 U.

The beneficial effects of the present invention are as follows.

The 32 kinds of Cap analog having a Cap2 structure in the present invention have a higher synthetic efficiency compared to the existing Cap analogs ARCA and Cleancap,

The 32 kinds of Cap analog having a Cap2 structure in the present invention have higher capping efficiency compared to the existing Cap analogs ARCA and Cleancap,

The 32 kinds of Cap analog having a Cap2 structure in the present invention show lower immunogenicity compared to the existing Cap analogs ARCA and Cleancap, and

The 32 kinds of Cap analog having a Cap2 structural in the present invention have higher protein translation efficiency compared to the existing Cap analog ARCA and Cleancap.

### Brief description of the drawings

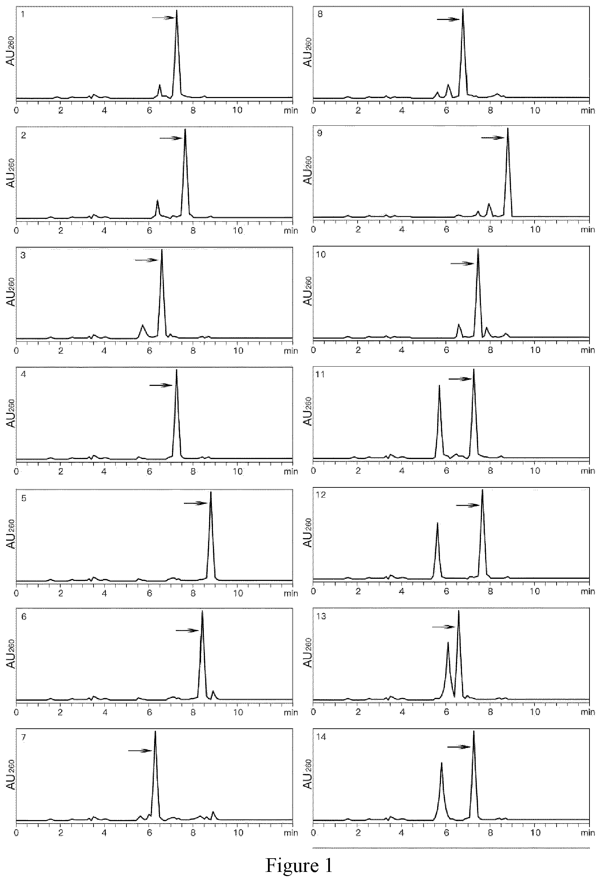

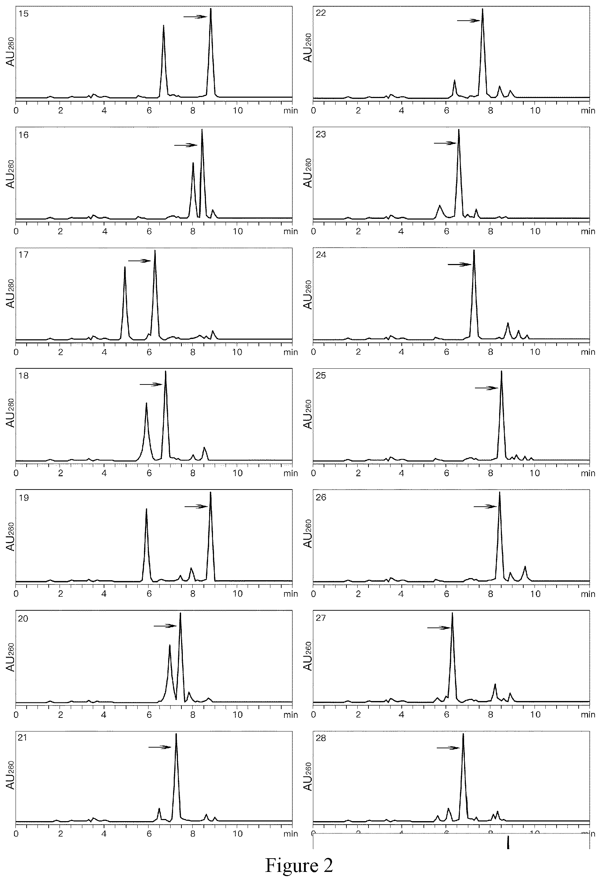

In Figures 1-3, reversed-phase high performance liquid chromatography (RP - HPLC) was used to verify the structure and homogeneity of each final product at 25 °C.

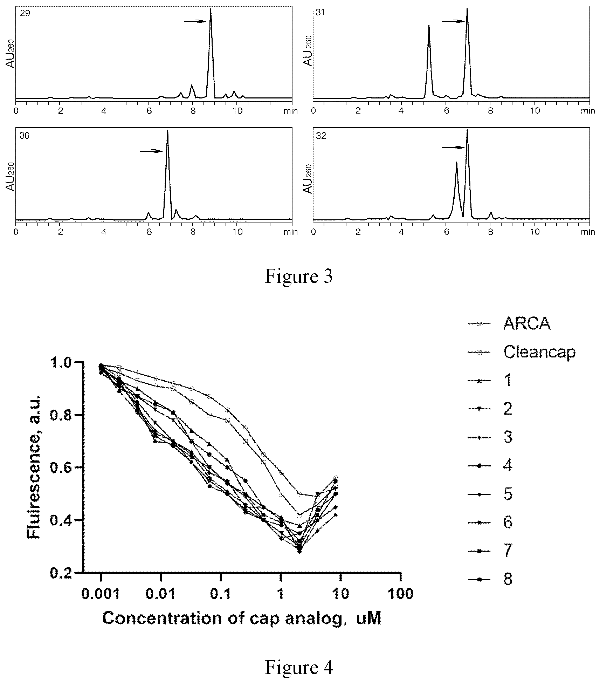

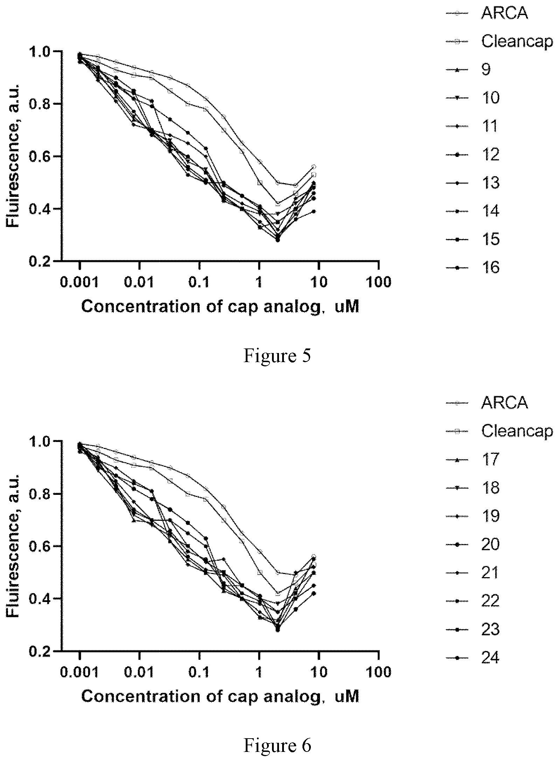

Figures 4-7 show the fluorescence titration curves for the products binding with Cap analogs. As the ligand concentration increases, the change in the curve indicates a weaker binding between eIF4E and the corresponding Cap analog. The increase in fluorescence signal with increasing ligand concentration is due to the increase in free Cap analog in the solution. Fluorescence intensity is expressed as relative values.

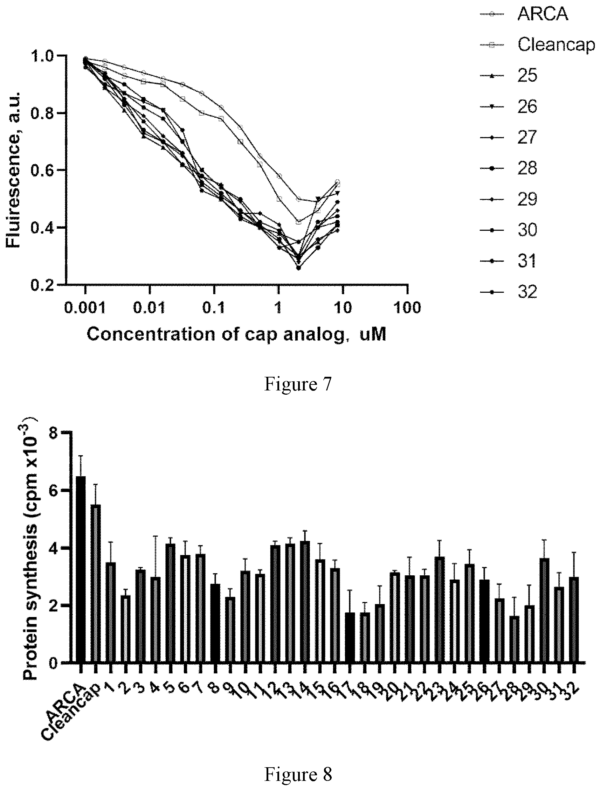

Figure 8 shows the effect of the Cap analogs on the inhibition of translation of rabbit reticulocyte lysate globin mRNA; natural rabbit globin mRNA was translated in the rabbit reticulocyte lysate system and the globin synthesis was detected by incorporating [³ H] Leu into the protein.

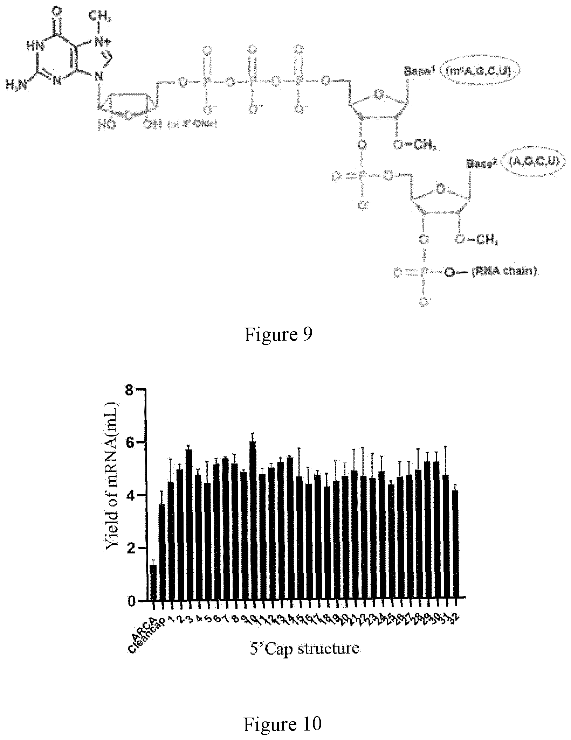

Figure 9 shows the general structural formula of the Cap analog provided by the present invention.
Figure 10 shows that the 32 kinds of the novel Cap analogs having a Cap2 structure provided by the present invention were applied to mRNA synthesis effectively increasing the efficiency of mRNA synthesis, and the synthesized products using the novel Cap analogs having a Cap2 structure (4-6 mg) were significantly higher than ARCA (1.5 mg) and Cleancap (3.8 mg) per ml of synthesis system.

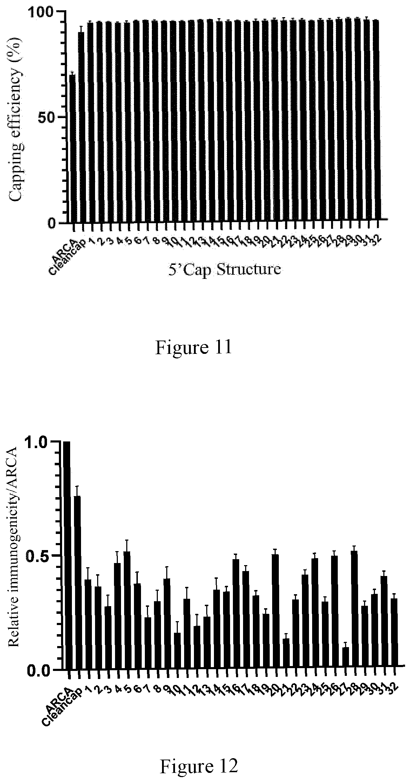

Figure 11 shows that the 32 kinds of the novel Cap analogs having a Cap2 structure provided by the present invention were applied to mRNA synthesis to effectively increase the capping efficiency of mRNA. And 95% of the synthesized products using the novel Cap analogs having a Cap2 structure were capped under the same synthesis conditions, higher than 70% for ARCA and 90% for Cleancap.
Figure 12 shows that the 32 kinds of the novel Cap analogs having a Cap2 structure provided by the present invention were applied to mRNA synthesis to effectively reduce immunogenicity, and that the intracellular immunogenicity of the synthesized products using the novel Cap analogs having a Cap2 structure was only 9%-52% of that of the same mRNA using the ACRA structure.

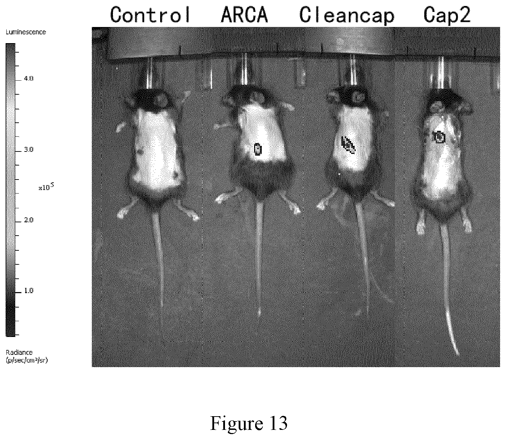

Figure 13 shows the novel Cap analogs having a Cap2 structure provided by the present invention applied to mRNA synthesis to effectively improve protein translation efficiency. mRNA encoding luciferase (Luc) was capped by ARCA, Cleancap and the novel Cap analogs having a Cap2 structure, respectively, and comparing the expression intensity of the three mRNAs in mice, it can be found that the expression intensity of Luc mRNA using the novel Cap analogs having a Cap2 structure was the highest.

### Detailed description

The present invention provides a novel 5'Cap analog having a Cap2 structure, wherein the novel 5'Cap analog having a Cap2 structure has a molecular formula selected from any one of the following:
m7G(5')ppp(5')(2'OMeA)p(2'OMeG),
m7G(5')ppp(5')(2'OMeG)p(2'OMeG),
m7G(5')ppp(5')(2'OMeC)p(2'OMeG),
m7G(5')ppp(5')(2'OMeU)p(2'OMeG),
m7G(5')ppp(5')(2'OMeA)p(2'OMeA),
m7G(5')ppp(5')(2'OMeG)p(2'OMeA),
m7G(5')ppp(5')(2'OMeC)p(2'OMeA),
m7G(5')ppp(5')(2'OMeU)p(2'OMeA),
m7G(5')ppp(5')(2'OMeA)p(2'OMeC),
m7G(5')ppp(5')(2'OMeG)p(2'OMeC),
m7G(5')ppp(5')(2'OMeC)p(2'OMeC),
m7G(5')ppp(5')(2'OMeU)p(2'OMeC),
m7G(5')ppp(5')(2'OMeA)p(2'OMeU),
m7G(5')ppp(5')(2'OMeG)p(2'OMeU),
m7G(5')ppp(5')(2'OMeC)p(2'OMeU),
m7G(5')ppp(5')(2'OMeU)p(2'OMeU),
3'-O-Me-m7G(5')ppp(5')(2'OMeA)p(2'OMeG),
3'-O-Me-m7G(5')ppp (5')(2'OMeG)p(2'OMeG),
3'-O-Me-m7G(5')ppp(5')(2'OMeC)p(2'OMeG),
3'-O-Me-m7G(5')ppp(5')(2'OMeU)p(2'OMeG),
3'-O-Me-m7G(5')ppp(5')(2'OMeA)p(2'OMeA),
3'-O-Me-m7G(5')ppp(5')(2'OMeG)p(2'OMeA),
3'-O-Me-m7G(5')ppp(5')(2'OMeC)p(2'OMeA),
3'-O-Me-m7G(5')ppp(5')(2'OMeU)p(2'OMeA),
3 -O-Me-m7G(5')ppp(5')(2'OMeA)p(2'OMeC),
3'-O-Me-m7G(5')ppp(5')(2'OMeG)p(2'OMeC),
3'-O-Me-m7G(5')ppp(5')(2'OMeC)p(2'OMeC),
3'-O-Me-m7G(5')ppp(5')(2'OMeU)p(2'OMeC),
3'-O-Me-m7G(5')ppp(5')(2'OMeA)p(2'OMeU),
3'-O-Me-m7G(5')ppp(5')(2'OMeG)p(2'OMeU),
3'-O-Me-m7G(5')ppp(5')(2'OMeC)p(2'OMeU), and
3'-O-Me-m7G(5')ppp(5')(2'OMeU)p(2'OMeU).

In the present invention, the commonality of the novel 5'Cap analog having a Cap2 structure described is based on methoxy modification of 2'OMe, methoxy modification of A\G\C\U and m7G nucleosides, namely, 2'OMeA or 2'OMeG or 2'OMeC or 2'OMeU, and 3'-O-Me-m7G. The novel Caps having a Cap2 structure are formed by enzymatic reactions.

The present invention also provides a method for the preparation of a novel 5'Cap analog having a Cap2 structure as described in the above technical solution, comprising the steps of
1) dissolving 2'-O-Methyl-ATP, 2'-O-Methyl-GTP, 2'-O-Methyl-CTP and 2'-O-Methyl-UTP in RNase-free water respectively, mixing with phosphate hydrolase and 2× Reaction Buffer respectively, and followed by incubation to obtain 2'-O-Methyl-ADP, 2'-O-Methyl-GDP, 2'-O-Methyl-CDP and 2'-O-Methyl-UDP;
2) dissolving the 2'-O-Methyl-ADP, 2'-O-Methyl-GDP, 2'-O-Methyl-CDP and 2'-O-Methyl-UDP obtained from the step 1) in RNase-free water respectively, mixing with 7-Methylguanosine, 7-Methyl-3'-O Methylguanosine, guanosyltransferase and 2× Reaction Buffer respectively, followed by incubation to obtain m7G(5')ppp(5')(2'OMeA/G/C/U) and 3'-O-Me-m7G(5')ppp(5')(2'OMeA/G/C/LT); and
3) dissolving the m7G(5')ppp(5')(2'OMeA/G/C/LT) and 3'-O-Me-m7G(5')ppp(5')(2'OMeA/G/C/U) obtained from the step 2) in RNase-free water respectively, mixing with T4 RNALigase1, then mixing with 2'-O-Methyl-ATP, 2'-O-Methyl-GTP, 2'-O-Methyl-CTP and 2'-O-Methyl-UTP respectively, followed by the mixing with 2×T4 RNA Ligase Reaction Buffer and incubation to obtain the novel 5'Cap analog having a Cap2 structure.

In the present invention, 2'-O-Methyl-ATP, 2'-O-Methyl-GTP, 2'-O-Methyl-CTP and 2'-O-Methyl-UTP were dissolved in RNase-free water respectively, mixed with phosphate hydrolase and 2× Reaction Buffer respectively, and followed by incubation to obtain 2'-O-Methyl-ADP, 2'-O-Methyl-GDP, 2'-O-Methyl-CDP and 2'-O-Methyl-UDP. In the present invention, the 2'-O-Methyl-ATP, 2'-O-Methyl-GTP, 2'-O-Methyl-CTP and 2'-O-Methyl-UTP are commercially available products.

In the present invention, the ratio of the amount of the 2'-O-Methyl-ATP, 2'-O-Methyl-GTP, 2'-O-Methyl-CTP and 2'-O-Methyl-UTP to the volume of RNase-free water is preferably all from 1 to 30 mmol:14 µL, more preferably, 10 mmol:14 µL. 2'-O-Methyl-ATP, 2'-O-Methyl-GTP, 2'-O-Methyl-CTP and 2'-O-Methyl-UTP are not specifically limited in source, they can be obtained as commercially available products or prepared by conventional methods. In the present invention, the volume ratio of the RNase-free water to phosphate hydrolase and 2× Reaction Buffer is preferably 14:1:15. In the present invention, the enzymatic activity of the phosphate hydrolase is preferably 50,000 U; the components of the 2× Reaction Buffer preferably comprise: 50 mM Tris-HCl, 5 mM KCl, 1 mM MgCl₂ andImM DTT, with pH value of 8. In the present invention, the incubation temperature is preferably 37 °C and the incubation time is preferably 1 h. In the present invention, the 2'-O-Methyl-ATP, 2'-O-Methyl-GTP, 2'-O-Methyl-CTP and 2'-O-Methyl-UTP are preferably purified by HPLC and then dissolved in RNase-free water.

In the present invention, the obtained 2'-O-Methyl-ADP, 2'-O-Methyl-GDP, 2'-O-Methyl-CDP and 2'-O-Methyl-UDP were dissolved in RNase-free water respectively, mixed with 7-Methylguanosine, 7-Methyl-3'-O Methylguanosine, guanosyltransferase and 2× Reaction Buffer respectively, and incubated to obtain m7G(5')ppp(5')(2'OMeA/G/C/U) and 3'-O-Me-m7G(5')ppp(5')(2'OMeA/G/C/U).

In the present invention, the volume ratio of the RNase-free water to 7-Methylguanosine, 7-Methyl-3'-O-Methylguanosine, guanosyltransferase, and 2× Reaction Buffer is preferably 11:10:10:1:22. In the present invention, the enzymatic activity of the guanosyltransferase is preferably 50,000 U. In the present invention, the components of the 2× Reaction Buffer preferably comprise: 50 mM Tris-HCl, 5 mM KCl, 1 mM MgCl₂ and 1 mM DTT, with pH value of 8. The source of the 7-Methylguanosine and 7-Methyl-3'-O-Guanosine is not specially restricted in the present invention, which are conventional commercially available or can be prepared by conventional preparation methods. In the present invention, the temperature of the incubation is preferably 37 °C and the time of the incubation is preferably 1h.

In the present invention, the obtained m7G(5')ppp(5')(2'OMeA/G/C/U) and 3'-O-Me-m7G(5')ppp(5')(2'OMeA/G/C/LT) were dissolved in RNase-free water respectively, mixed with T4 RNA Ligase 1 and then mixed with 2'-O-Methyl-ATP, 2'-O-Methyl-ATP and 2'-O-Methyl-UTP respectively, followed by mixing with 2×T4 RNA Ligase Reaction Buffer and incubation to obtain the novel Cap2 structural 5'Cap analogs.

In the present invention, the m7G(5')ppp(5')(2'OMeA/G/C/U) and 3'-O-Me-m7G(5')ppp(5')(2'OMeA/G/C/U) are preferably purified by HPLC and then dissolved in RNase-free water.

In the present invention, the volume ratio of RNase-free water to T4 RNA Ligase 1 and 2×T4 RNA Ligase Reaction Buffer is preferably 11:1:22. In the present invention, the T4 RNA Ligase 1 has an enzymatic activity of preferably 10,000 U. In the present invention, the components of the 2×T4 RNA Ligase Reaction Buffer preferably comprise: 60 mM Tris-HCl, 20 mM MgCl₂, 20 mM DTT and 2 mM ATP. In the present invention, the incubation temperature is preferably 37 °C and the time of the incubation is preferably 1h.

The technical solutions provided by the present invention are described in detail below in connection with the embodiments, but they should not be used to limit the protection scope of the present invention.

### Example 1

1. 10 mmol of 2'-O-Methyl-ATP, 2'-O-Methyl-GTP, 2'-O-Methyl-CTP or 2'-O-Methyl-UTP were dissolved in RNase-free water, respectably, in a total volume of 14 µL. After adding 1 µL (50,000 U) of phosphate hydrolase and 15 µL of 2× Reaction Buffer (50 mM Tris-HCl, 5 mM KCl, 1 mM MgCl₂, 1 mM DTT, with pH value of 8), the mixture was incubated for 1 h at 37 °C to obtain 2'-O-Methyl-ADP, 2'-O-Methyl-GDP, 2'-O-Methyl-CDP and 2'-O-Methyl-UDP.
2. The above obtained 2'-O-Methyl-ADP, 2'-O-Methyl-GDP, 2'-O-Methyl-CDP and 2'-O-Methyl-UDP were purified by HPLC, respectively and dissolved in RNase-free water in a total volume of 11 µL.
3. 10 µL (10 mmol) 7-Methylguanosine, 10 µL (10 mmol) 7-Methyl-3'-O-Methylguanosine and 1 µL (50,000 U) guanosyltransferase were added to the solution obtained in the step 2. Adding 22 µL 2× Reaction Buffer (50 mM Tris-HCl, 5 mM KCl, 1 mM MgCl₂ and 1 mM DTT, with pH value of 8) and the mixture was incubated for 1h at 37 °C to obtain m7G(5')ppp(5') (2'OMeA/G/C/LT) or 3'-O-Me-m7G(5')ppp(5') (2'OMeA/G/C/U).
4. The solution obtained in step 3 was purified by HPLC and dissolved in RNase-free water and set aside, with a total volume of 11 µL.
5. 10 µL (10 mmol) of 2'-O-Methyl-ATP, 2'-O-Methyl-GTP, 2'-O-Methyl-CTP or 2'-O-Methyl-UTP were added to the solution obtained in step 4 with 1 µL (10,000 U) of T4 RNA ligase 1 (ssRNA ligase NEB M0437M), respectively. After adding 22 µL of 2× T4 RNA Ligase Reaction Buffer (60 mM Tris-HCl (pH 7.8 at 25°C), 20 mM MgCl₂, 20 mM DTT and 2 mM ATP), the mixture was incubated for 1h at 37 °C to obtain the novel 5'Cap analog having a Cap2 structure.
6. The solution obtained in step 5 was purified by HPLC and dissolved in RNase-free water as follows:
   1) RP HPLC experimental parameters were set to the flow rate of 1 mL/min (chromatographic column with an inner diameter of 4.6 mm); 0.1 mL/min (chromatographic column with an inner diameter of 2.1 mm);
   2) Injecting 100 µL of sample (0.1 mgL/ml) (namely, the amount of each Cap analog to be measured was 10 µg, where 1 µg = 100 mAU at a flow rate of 1 mL/min ); for a chromatographic column with 2.1 mm, adding 10 µL of standard protein with a concentration of 0.1 mg/mL, that is the amount of each Cap analog to be measured was 1 µg (1 µg = 100 mAU at a flow rate of 0.1 mL/min); and
   3) For sample collection, eluted peaks were collected in capped polypropylene microcentrifuge tubes, tightly capped and stored at -20 °C.

The molecular formulas are as follows:
1. m7G(5')ppp(5')(2'OMeA)p(2'OMeG),
2. m7G(5')ppp(5')(2'OMeG)p(2'OMeG),
3. m7G(5')ppp(5')(2'OMeC)p(2'OMeG),
4. m7G(5')ppp(5')(2'OMeU)p(2'OMeG),
5. m7G(5')ppp(5')(2'OMeA)p(2'OMeA),
6. m7G(5')ppp(5')(2'OMeG)p(2'OMeA),
7. m7G(5')ppp(5')(2'OMeC)p(2'OMeA),
8. m7G(5')ppp(5')(2'OMeU)p(2'OMeA),
9. m7G(5')ppp(5')(2'OMeA)p(2'OMeC),
10. m7G(5')ppp(5')(2'OMeG)p(2'OMeC),
11. m7G(5')ppp(5')(2'OMeC)p(2'OMeC),
12. m7G(5')ppp(5')(2'OMeU)p(2'OMeC),
13. m7G(5')ppp(5')(2'OMeA)p(2'OMeU),
14. m7G(5')ppp(5')(2'OMeG)p(2'OMeU),
15. m7G(5')ppp(5')(2'OMeC)p(2'OMeU),
16. m7G(5')ppp(5')(2'OMeU)p(2'OMeU),
17. 3'-O-Me-m7G(5')ppp(5')(2'OMeA)p(2'OMeG),
18. 3'-O-Me-m7G(5')ppp (5')(2'OMeG)p(2'OMeG),
19. 3'-O-Me-m7G(5')ppp(5')(2'OMeC)p(2'OMeG),
20. 3'-O-Me-m7G(5')ppp(5')(2'OMeU)p(2'OMeG),
21. 3'-O-Me-m7G(5')ppp(5')(2'OMeA)p(2'OMeA),
22. 3'-O-Me-m7G(5')ppp(5')(2'OMeG)p(2'OMeA),
23. 3'-O-Me-m7G(5')ppp(5')(2'OMeC)p(2'OMeA),
24. 3'-O-Me-m7G(5')ppp(5')(2'OMeU)p(2'OMeA),
25. 3'-O-Me-m7G(5')ppp(5')(2'OMeA)p(2'OMeC),
26. 3'-O-Me-m7G(5')ppp(5')(2'OMeG)p(2'OMeC),
27. 3'-O-Me-m7G(5')ppp(5')(2'OMeC)p(2'OMeC),
28. 3'-O-Me-m7G(5')ppp(5')(2'OMeU)p(2'OMeC),
29. 3'-O-Me-m7G(5')ppp(5')(2'OMeA)p(2'OMeU),
30. 3'-O-Me-m7G(5')ppp(5')(2'OMeG)p(2'OMeU),
31. 3'-O-Me-m7G(5')ppp(5')(2'OMeC)p(2'OMeU), and
32. 3'-O-Me-m7G(5')ppp(5')(2'OMeU)p(2'OMeU).

The prepared 32 kinds of the novel Cap analogs having a Cap2 structure were identified using RP HPLC at 25 °C. The results are shown in Figures 1-3.

### Example 2

By testing the binding ability of 32 kinds of the novel Cap analogs having a Cap2 structure prepared in Example 1 to eIF4E, it was concluded that the higher the binding ability, the stronger the translational activity.

The binding ability of Cap analogs to eIF4E was examined. Eukaryotic initiation factor 4E (eIF 4 E) is a Cap-binding protein that specifically recognizes the Cap structure at the 5' end of mRNA and plays an important role in the initiation of eukaryotic translation. Due to the presence of tryptophan, eIF4E itself can be excited at a wavelength of 280 nm and fluorescence is detected at a wavelength of 337 nm. After binding to the Cap structure, the fluorescence diminishes, so the present method serves as a standard for detecting the binding ability of the Cap structure to elF4E. The results are shown in Figure 4-7.

Experimental method: 1 µL of different concentrations of Cap structure solution was added dropwise into 1.4 mL of 0.1 µM eIF4E protein (solvent: 50 mM HEPES/KOH (pH 7.2), 100 mM KCl, 0.5 mM EDTA and 1 mM DTT), and the fluorescence change was detected at a wavelength of 337 nm with excitation at a wavelength of 280 nm.

### Example 3

The biological activity of the 32 kinds of the novel Cap analogs having a Cap2 structure prepared in Example 1 was determined by testing the effect of the Cap analogs having a Cap2 structure on globin expression levels.

Micrococcus nuclease treatment of rabbit reticulocyte lysates removes nucleic acids and serves as an in vitro translation system for mRNA in which mRNA can be expressed. Natural rabbit globulin mRNA was added to the system at a final concentration of 5 ug/mL along with ³H-labeled leucine. 100 µmol Cap analogs having a Cap2 structure (ARCA and Cleancap as controls) were added to each 1mL of the reaction system, and the proteins in the reaction system were extracted after 24 hours and assayed for globulin ³H content.

Because the Cap analogs compete for binding to eIF4E, the stronger the incorporated Cap analogs bind to eIF4E, the lower the translation level of the incorporated natural mRNA and the lower ³H content of the globin. The results are shown in Figure 8.

Experimental method: Micrococcus nuclease treatment of rabbit reticulocyte lysates to remove nucleic acids was used as a reaction system for the mRNA in vitro translation system, in which mRNA can be expressed. Natural rabbit globulin mRNA was added to the system at a final concentration of 5 µg/ml, along with ³H-labeled leucine. 100 µmol of the cap analogs having a Cap2 structure (ARCA and Cleancap were used as controls) were added to each 1 ml of the reaction system, and the proteins in the reaction system were extracted after 24 h. The ³H content of the globulin was measured.

### Example 4

The 32 kinds of the novel Cap analogs having a Cap2 structure prepared in Example 1 were applied to mRNA synthesis, effectively increasing the efficiency of mRNA synthesis (4-6 mg/mL).

### Experimental methods:

1. Before synthesizing mRNA, linearize the plasmid with NotI, digested at 4 °C overnight;
2. Extract with DNA template;
3. In vitro transcriptional synthesize mRNA, using ARCA, Cleancap and the 32 kinds of Cap2 in the present invention as Cap structures respectively. The reaction systems are shown in Table 1;

**Table 1 Reaction system**

| Component | Dosage |
|---|---|
| T7 10X Reaction Buffer | 2µl |
| T7 ATP Solution (75 mM) | 2µl |
| T7 CTP Solution (75 mM) | 2µl |
| T7 GTP Solution (75 mM) | 2µl |
| T7 UTP Solution (75 mM) | 2µl |
| Linearized plasmid templates | <8µl |
| T7 Enzyme Mix | 2µl |
| ARCA/Cleancap/Cap2 | 2µl |
| Nuclease-free water | to 20µl |

React at 37 °C for 6 h. Digest with TURBO DNase for 15 min.
4. Purify the transcription products using LiCl solution and calculate the reaction yields.

The results are shown in Table 2 and Figure 10. Up to 1.5 mg mRNA per ml of the transcription reaction system was prepared using ARCA Cap structure, up to 3.8 mg mRNA per ml of the transcription reaction system was prepared using Cleancap, and up to 4 ~ 6 mg mRNA per ml of the transcription reaction system was prepared using Cap2 in the present invention.

**Table 2 Mass of final product obtained from 1 ml mRNA synthesis reaction system (mg)**

| ARCA | Cleancap | 1 | 2 | 3 | 4 | 5 | 6 | 7 |
|---|---|---|---|---|---|---|---|---|
| 1.5 | 3.8 | 4.5 | 4.9 | 5.8 | 4.7 | 4.4 | 5.1 | 5.3 |
| 8 | 9 | 10 | 11 | 12 | 13 | 14 | 15 | 16 |
| 5.3 | 4.8 | 6.0 | 4.7 | 4.9 | 5.1 | 5.5 | 4.6 | 4.2 |
| 17 | 18 | 19 | 20 | 21 | 22 | 23 | 24 | 25 |
| 4.7 | 4.1 | 4.4 | 4.6 | 4.8 | 4.6 | 4.5 | 4.7 | 4.1 |
| 26 | 27 | 28 | 29 | 30 | 31 | 32 | -- | -- |
| 4.6 | 4.7 | 4.8 | 5.3 | 5.3 | 4.5 | 4.3 | -- | -- |

### Example 5

The 32 kinds of the novel Cap analogs having a Cap2 structure prepared in Example 1 were applied to mRNA synthesis to effectively increase capping efficiency (95-98%).

### Experimental methods:

The mRNA molecules with different Cap structures obtained in Example 1 were subjected to enzymatic cleavage, and the ratio of capped and uncapped mRNA molecules could be determined in a liquid chromatography-mass spectrometry analysis experiment.

### Beads pre-processing:

1. Concentrate the beads using a magnet, aspirate the supernatant storage solution, and add an equal volume of 0.1 M NaOH + 0.05 M NaCl;
2. Aspirate the supernatant and resuspend the beads with an equal volume of 0.1 M NaCl, keep the concentration constant;
3. Anneal mRNA and cleavage tags, configure the following reaction system;

**Table 3 Reaction system**

| | |
|---|---|
| 10× RNase H reaction buffer | 12µl |
| RNase H probe | 500 pmol |
| mRNA | 100 pmol |
| total | 120µl |

Gradient annealing: 95°C 5min
65°C 2min
55°C 2min
22°C hold

4. Combining cleavage tags with beads
Beads were centrifuged at 15,000g for 5min, and the supernatant was removed, then 120µl of annealed mRNA and tags were added and incubated for 30min at room temperature, during which time they were gently shaken to ensure adequate binding.
5. Enzyme cleavage using RNase H;
Add 10 µl RNase H and incubate at 37 °C for 3 h. Precipitate the beads with magnet and discard the supernatant.
Add 100 µl washing buffer, mix thoroughly, magnet precipitate for 2 min, and discard the supernatant. Repeat 3 times.
Wash with DI water for 3 times.

6. Hydrolysis with RppH;
Use 10 units RppH in nebuffer 2 to hydrolyze at room temperature for 1h.
7. Wash with DI water for 3 times;
8. Elute;
DI water was aspirated, 100 µl of 75% ethanol preheated to 80 °C was added, incubat for 3 min, and magnet precipitat for 3 min. The supernatant was taken, followed by evaporating, and centrifuge for 45 min to a volume of 10 µl. Finally, the sample was resuspended in 50 µl of 100 M EDTA/1% MeOH and left for LC-MS analysis.
9. the mRNA capping rate of different Cap analogs synthesized was analyzed with LC-MS.

The results are shown in Table 4 and Figure 11. The maximum capping rate of transcription product was 70% when using the ARCA Cap structure, the maximum capping rate of transcription product is 90% when Cleancap is used, and the maximum capping rate of transcription product is up to 95% when Cap2 in the present invention is used.

**Table 4 mRNA capping rates when using different Cap structures**

| ARCA | Cleancap | 1 | 2 | 3 | 4 | 5 | 6 | 7 |
|---|---|---|---|---|---|---|---|---|
| 70% | 90% | 94.91% | 95.1% | 94.82% | 94.72% | 94.88% | 94.65% | 94.76% |
| 8 | 9 | 10 | 11 | 12 | 13 | 14 | 15 | 16 |
| 94.79% | 94.73% | 94.93% | 95.2% | 94.86% | 94.77% | 94.82% | 94.83% | 94.84% |
| 17 | 18 | 19 | 20 | 21 | 22 | 23 | 24 | 25 |
| 94.91% | 94.98% | 95% | 94.42% | 94.55% | 94.83% | 94.65% | 94.72% | 94.8% |
| 26 | 27 | 28 | 29 | 30 | 31 | 32 | - | - |
| 94.97% | 94.88% | 94.62% | 94.93% | 94.8% | 94.7% | 94.66% | - | - |

### Example 6

The 32 kinds of the novel Cap analogs having a Cap2 structure prepared in Example 1 were applied to mRNA synthesis to effectively reduce immunogenicity.

Experimental methods: the intracellular immunoproteins TLR3, TLR7, TLR8 and RIG-1 were immunoprecipitated together with their bound RNAs by RNA immunoprecipitation, and finally these mRNAs were identified by real-time quantitative PCR, and their relative abundance was proportional to their immunogenicity.

### Experimental methods:

1. Cell collection
   Human-derived PBMC cells were cultured to 10⁶ cells/well and cells were transfected with 5 µg of mRNA having ARCA, mRNA having Cleancap and mRNA having Cap 2 structure using Lipofectamine 2000, respectively. The cells were collected by trypsin digestion 24 h later and resuspended in PBS, centrifuged, and collected.
2. Cell fixation and lysis
   Fixative was added to the cells, and after 15 minutes glycine was added for termination of fixation, followed by centrifugation to collect the cells. Cells were resuspended by adding lysate, incubated on ice at 4°C for 30 min, centrifuged at 2400g for 10 min, and the supernatant was collected.
3. RNA Immunoprecipitation
   TLR3, TLR7, TLR8 and RIG-1 antibodies (2-10 µg) were added to the supernatant (6-10 mg) followed by gentle incubation at 4°C for 2 hours. Protein A/G beads (40 µL) were then added to them and incubated gently for 1 h at 4 °C.
4. Washing away the unbound material
   Centrifuge at 2500 rpm for 30 seconds with precipitated magnetic beads and remove the supernatant. Resuspend the beads in 500 µL of RIP buffer. Wash in RIP buffer a total of three times, followed by one wash in PBS.
5. Purification of RNA bound on RBP after immunoprecipitation Resuspend magnetic beads in TRIzol RNA Extraction Reagent (1 mL) and isolate co-precipitated RNA, and then elute RNA using nuclease-free water (20 µL).
6. Reverse transcribe (RT) the RNA into cDNA and design primers according to the mRNA sequence for quantitative PCR analysis. mRNA immunogenicity is proportional to the number of mRNA copies precipitated by magnetic beads.

The results are shown in Table 5 and Figure 12. The immunogenicity of mRNA containing the Cap having a Cap2 structure in the present invention is significantly lower than that of the Cap having an ARCA structure and the Cap having a Cleancap Cap.

Table 5 Intracellular immunogenicity of mRNA when using different Cap structures, all results are relative to ARCA.

**Table 5 Results of mRNA immunogenicity assay for the Caps having the Cap2 structure**

| ARCA | Cleancap | 1 | 2 | 3 | 4 | 5 | 6 | 7 |
|---|---|---|---|---|---|---|---|---|
| 1 | 0.76 | 0.40 | 0.37 | 0.28 | 0.48 | 0.52 | 0.38 | 0.24 |
| 8 | 9 | 10 | 11 | 12 | 13 | 14 | 15 | 16 |
| 0.31 | 0.4 | 0.18 | 0.31 | 0.19 | 0.23 | 0.35 | 0.34 | 0.48 |
| 17 | 18 | 19 | 20 | 21 | 22 | 23 | 24 | 25 |
| 0.44 | 0.31 | 0.26 | 0.51 | 0.15 | 0.29 | 0.41 | 0.48 | 0.29 |
| 26 | 27 | 28 | 29 | 30 | 31 | 32 | -- | -- |
| 0.49 | 0.09 | 0.52 | 0.27 | 0.31 | 0.4 | 0.3 | -- | -- |

### Example 7

The effective protein translation efficiency of the novel Cap analogs having a Cap2 structure prepared in Example 1 when applied to mRNA synthesis.

### Experimental method:

An equal amount of mRNA encoding luciferase was injected intradermally into the back of mice, and luciferase substrate was injected into the tail vein 24h later, and the luminescence intensity was proportional to the effective target protein translation rate.

The experimental results are shown in Figure 13. The effective protein translation efficiency of the novel Cap analogs having a Cap2 structure in the present method is significantly higher than that of the ARCA and Cleancap Cap structures when applied to mRNA synthesis.

The above described is only the preferred embodiments of the present invention, and it should be noted that for a person of skilled in the art, other improvements and embellishments can be made without departing from the principles of the present invention, and these improvements and embellishments should also be considered as the scope of protection of the present invention.

## Claims

1. A novel 5'Cap analog having a Cap2 structure, **characterized in that** the novel 5'Cap analog having a Cap2 structure has a molecular formula selected from any one of the following:
m7G(5')ppp(5')(2'OMeA)p(2'OMeG),
m7G(5')ppp(5')(2'OMeG)p(2'OMeG),
m7G(5')ppp(5')(2'OMeC)p(2'OMeG),
m7G(5')ppp(5')(2'OMeU)p(2'OMeG),
m7G(5')ppp(5')(2'OMeA)p(2'OMeA),
m7G(5')ppp(5')(2'OMeG)p(2'OMeA),
m7G(5')ppp(5')(2'OMeC)p(2'OMeA),
m7G(5')ppp(5')(2'OMeU)p(2'OMeA),
m7G(5')ppp(5')(2'OMeA)p(2'OMeC),
m7G(5')ppp(5')(2'OMeG)p(2'OMeC),
m7G(5')ppp(5')(2'OMeC)p(2'OMeC),
m7G(5')ppp(5')(2'OMeU)p(2'OMeC),
m7G(5')ppp(5')(2'OMeA)p(2'OMeU),
m7G(5')ppp(5')(2'OMeG)p(2'OMeU),
m7G(5')ppp(5')(2'OMeC)p(2'OMeU),
m7G(5')ppp(5')(2'OMeU)p(2'OMeU),
3'-O-Me-m7G(5')ppp(5')(2'OMeA)p(2'OMeG),
3'-O-Me-m7G(5')ppp (5')(2'OMeG)p(2'OMeG),
3'-O-Me-m7G(5')ppp(5')(2'OMeC)p(2'OMeG),
3'-O-Me-m7G(5')ppp(5')(2'OMeU)p(2'OMeG),
3'-O-Me-m7G(5')ppp(5')(2'OMeA)p(2'OMeA),
3'-O-Me-m7G(5')ppp(5')(2'OMeG)p(2'OMeA),
3'-O-Me-m7G(5')ppp(5')(2'OMeC)p(2'OMeA),
3'-O-Me-m7G(5')ppp(5')(2'OMeU)p(2'OMeA),
3 -O-Me-m7G(5')ppp(5')(2'OMeA)p(2'OMeC),
3'-O-Me-m7G(5')ppp(5')(2'OMeG)p(2'OMeC),
3'-O-Me-m7G(5')ppp(5')(2'OMeC)p(2'OMeC),
3'-O-Me-m7G(5')ppp(5')(2'OMeU)p(2'OMeC),
3'-O-Me-m7G(5')ppp(5')(2'OMeA)p(2'OMeU),
3'-O-Me-m7G(5')ppp(5')(2'OMeG)p(2'OMeU),
3'-O-Me-m7G(5')ppp(5')(2'OMeC)p(2'OMeU), and
3'-O-Me-m7G(5')ppp(5')(2'OMeU)p(2'OMeU).

2. A method of preparing the novel 5'Cap analog having a Cap2 structure of claim 1, **characterized in that** the method comprises the steps of
1) dissolving 2'-O-Methyl-ATP, 2'-O-Methyl-GTP, 2'-O-Methyl-CTP and 2'-O-Methyl-UTP in RNase-free water respectively, mixing with phosphate hydrolase and 2× Reaction Buffer respectively, and followed by incubation to obtain 2'-O-Methyl-ADP, 2'-O-Methyl-GDP, 2'-O-Methyl-CDP and 2'-O-Methyl-UDP;
2) dissolving the 2'-O-Methyl-ADP, 2'-O-Methyl-GDP, 2'-O-Methyl-CDP and 2'-O-Methyl-UDP obtained from the step 1) in RNase-free water respectively, mixing with 7-Methylguanosine, 7-Methyl-3'-O-Methylguanosine, guanosyltransferase and 2× Reaction Buffer respectively, and followed by incubation to obtain m7G(5')ppp(5')(2'OMeA/G/C/U) and 3'-O-Me-m7G(5')ppp(5')(2'OMeA/G/C/LT); and
3) dissolving the m7G(5')ppp(5')(2'OMeA/G/C/LT) and 3'-O-Me-m7G(5')ppp(5')(2'OMeA/G/C/U) obtained from the step 2) in RNase-free water respectively, mixing with T4 RNA Ligase1, then mixing with 2'-O-Methyl-ATP, 2'-O-Methyl-GTP, 2'-O-Methyl-CTP and 2'-O-Methyl-UTP respectively, followed by mixing with 2×T4 RNA Ligase Reaction Buffer and incubation to obtain the novel 5'Cap analog having a Cap2 structure.

3. The preparation method according to claim 2, **characterized in that** the ratio of the amount of 2'-O-Methyl-ATP, 2'-O-Methyl-GTP, 2'-O-Methyl-CTP and 2'-O-Methyl-UTP in the step 1) to the volume of RNase-free water is 1 to 30 mmol:14 µL.

4. The preparation method according to claim 2, **characterized in that** the volume ratio of RNase-free water to phosphate hydrolase and 2× Reaction Buffer in the step 1) is 14:1:15.

5. The preparation method according to claim 4, **characterized in that** the phosphate hydrolase has an enzymatic activity of 50,000 U, and
the components of the 2× Reaction Buffer comprise: 50 mM Tris-HCl, 5 mM KCl, 1 mM MgCl₂ and 1 mM DTT, with pH value of 8.

6. The preparation method according to claim 2, **characterized in that** the incubation temperature of steps 1) to 3) is 37 °C respectively, and the incubation time thereof is 1h respectively.

7. The preparation method according to claim 2, **characterized in that** in the step 2), the volume ratio of RNase-free water to 7-Methyl-Guanosine, 7-Methyl-3'-O-Methyl-Guanosine, guanosyltransferase and 2× Reaction Buffer is 11:10:10:1:22.

8. The preparation method according to claim 3, **characterized in that** the volume ratio of RNase-free water to T4 RNA Ligase 1 and 2× T4 RNA Ligase Reaction Buffer is 11:1:22.

9. The preparation method according to claim 2 or 8, **characterized in that**, the components of the 2×T4 RNA Ligase Reaction Buffer comprise: 60 mM Tris-HCl, 20 mM MgCl₂, 20 mM DTT and 2 mM ATP.

10. The preparation method according to claim 2 or 8, **characterized in that** the enzyme activity of the T4 RNA Ligase 1 is 10,000 U.
